# EUROPÄISCHE PATENTSCHRIFT

(11) **EP 4 368 969 B1**
(45) Veröffentlichungstag und Bekanntmachung des Hinweises auf die Patenterteilung: **26.08.2026**
(21) Anmeldenummer: 23206363.6
(22) Anmeldetag: 27.10.2023
(51) Int. Cl.: G01N 21/3563, G01N 21/359, G01N 21/85, A01D 41/127

(54) **VERFAHREN UND ANORDNUNG ZUR ERMITTLUNG DES TAUSENDKORNGEWICHTS VON KÖRNERFRÜCHTEN**
METHOD AND ARRANGEMENT FOR DETERMINING THE THOUSAND-GRAIN WEIGHT OF GRAINS
PROCÉDÉ ET DISPOSITIF POUR LA DÉTERMINATION DU POIDS DE MILLE GRAINS DE CÉRÉALES

(30) Priorität: 11.11.2022 DE 102022129876
(43) Veröffentlichungstag der Anmeldung: 15.05.2024
(73) Patentinhaber: Deere & Company, Moline, IL 61265 (US)
(72) Erfinder: Schade, Peter, Mannheim (DE); Struve, Carsten, Mannheim (DE); Gralfs, Renke, Mannheim (DE)
(74) Vertreter: Holst, Sönke

(56) Entgegenhaltungen:
- EP-A1- 3 366 104
- CN-A- 112 304 947
- JP-A- 2023 125 301
- KR-A- 20160 037 507

## Beschreibung

Die vorliegende Erfindung bezieht sich auf ein Verfahren und eine Anordnung zur Ermittlung einer massen- und/oder größenspezifischen Größe der Körner von Körnerfrüchten.

### Stand der Technik

Die moderne Präzisions-Landwirtschaft ist bestrebt, die vorhandenen Ressourcen, wie Wasser, Boden, Saatgut und Düngemittel optimal zu nutzen, um die Ertragspotentiale möglichst gut auszuschöpfen, unter minimalem Verbrauch der Ressourcen. Hierbei werden im Stand der Technik bei der Ernte durch geeignete Messeinrichtungen unter gleichzeitiger Positionsbestimmung Ertragskarten erzeugt, um darauf basierend anhand der Erträge beispielsweise Düngermengen und Säraten ortsspezifisch zu variieren (WO 86/05353 A1). Eine Verfeinerung der Planung nachfolgender Maßnahmen der Präzisions-Landwirtschaft oder bei der Pflanzenzüchtung wird durch die Messung von Inhaltsstoffen, wie Stärke- und Proteingehalten, des Ernteguts ermöglicht (EP 2 189 781 A2, KR 2016 0037507 A, EP 3 366 104 A1).

Die bisherigen Vorgehensweisen messen die Erträge und ggf. Inhaltsstoffe des Ernteguts zwar ortsbezogen, jedoch bleibt bei der Ernte von Körnerfrüchten, wie Getreide oder Mais, ein wesentlicher Gesichtspunkt unberücksichtigt, nämlich die Abmessungen oder Masse der einzelnen Körner, die üblicherweise als so genannte Tausendkornmasse (bzw. Tausendkorngewicht) gemessen wird, bzw. die Anzahl der Körner je Flächeneinheit des Felds. Der im Stand der Technik bereits ortsspezifisch gemessene Ertrag, d.h. die Masse der Körner (im Folgenden werden mehrere Körner auch als "das Korn" bezeichnet) je Flächeneinheit des Feldes, kann durch Multiplikation der Anzahl der Körner je Flächeneinheit mit der (durch 1000 geteilten) Tausendkornmasse berechnet werden. Die Tausendkornmasse wird üblicherweise gemessen, indem man 1000 Körner abzählt und deren Masse misst (man könnte alternativ dazu das Volumen von 1000 Körnern mit deren Massendichte (Masse je Volumen) multiplizieren). Wenn beliebige zwei der agronomischen Größen Ertrag, Masse (bzw. Tausendkornmasse oder Volumen und Dichte der einzelnen Körner) der einzelnen Körner und Anzahl der Körner je Fläche bekannt sind, kann die dritte agronomische Größe berechnet werden. Bisher ist aber nur eine Messung des Ertrags vorgesehen.

Die Korngröße oder Tausendkornmasse oder Abmessungen der Körner enthalten wichtige Informationen für den Landwirt, denn zum Beispiel kann oder können eine hohe Tausendkornmasse bzw. relativ große Körner bei geringen Erträgen darauf hindeuten, dass sich die Erträge durch eine Erhöhung der Kornanzahl pro Fläche vergrößern ließen, da an dem betroffenen Standort mehr Ressourcen zur Verfügung stehen als von den geernteten Pflanzen genutzt wurden, während relativ kleine Tausendkornmassen bzw. relativ kleine Körner auf zu hohe Kornzahlen pro Fläche hindeuten, da an dem betroffenen Standort weniger Ressourcen zur Verfügung stehen als benötigt würden, um alle potentiell zur Reife kommenden Körner auch tatsächlich ausreifen zu lassen. Die Komponenten Kornzahl pro Fläche und Tausendkornmasse lassen sich aus mit Hilfe der Anpassung der Särate (Anlage Pflanzen pro Fläche) und der Frühjahrsdüngung (Beeinflussung der Bestockung und damit der Anzahl der Ähren/Kolben/Schoten pro Fläche und Anzahl Körner je Ähre/Kolben/Schote) beeinflussen. Mithilfe der Säate können die gesäten Körner pro Fläche beeinflusst werden, da mehr ausgesäte Körner zu mehr Halmen pro Fläche führen, was wiederum mehr Ähren/Kolben/Schoten und damit mehr Körner pro Fläche zur Folge hat. Dieser Prozess gilt ebenso umgekehrt.

Auch kann die Bestockung der Pflanze (bezeichnet das Stadium des Verzweigungswachstums) beeinflusst werden. Die Anzahl der gebildeten Ähren beim Getreide kann durch die Frühjahrsdüngung beeinflusst werden. Gestaltet man diese Düngung höher, bildet die Pflanze mehr Triebe, umgekehrt bei einer reduzierten Düngung weniger Triebe. Diese Triebe bilden im späteren Verlauf des Wachstums dann Ähren, bilden aber auch Blattmasse, die Wasser verdunstet und somit bei zu hoher Blattmasse eine frühzeitige Abreife bewirken kann.

Ebenfalls bestimmt die Frühjahrdüngung die Anzahl der Körner je Ähre, Kolben oder Schote. Über diesen Mechanismus kann also auch die Kornzahl pro Fläche beeinflusst werden, da mit mehr Trieben mehr Ähren und somit mehr Körner gebildet werden. Durch die Anzahl Körner pro Fläche verändert sich auch die Tausendkornmasse (je mehr Körner, desto geringer die Tausendkornmasse und umgekehrt, wenn Wasser begrenzender Faktor ist) Für jeden Standort gibt es ein optimales Verhältnis zwischen Kornzahl pro Fläche und der Tausendkornmasse. Aus diesen beiden Komponenten setzt sich der Ertrag zusammen. Bisher wird aber nur der Ertrag gemessen, ohne zu wissen wie die Komponenten Kornzahl und Tausendkornmasse sich verhalten und welcher Faktor limitierend gewesen sein kann beim Ertrag. Somit kann bisher keine Aussage über die korrekte Aussaatmenge oder eine passende Dosierung der Düngegaben getroffen werden, da sich einzelnen Komponenten des Ertrags nicht in der Fläche bestimmt haben lassen.

Bisher wurde die Tausendkornmasse - zu Zwecken der Verlust- oder Überkehrkornmengenanzeige - beim Mähdrescher als konstant angenommen (EP 1 516 522 A2, DE 34 20 800 A1) und ggf. vor der Ernte ausgezählt.

Zudem wurde eine Erfassung von Körnerabmessungen mittels einer Kamera und Bildverarbeitung zur Ertragsvorhersage an auf einem Feld wachsenden Pflanzen (WO 2018/073163 A1, WO 2018/073093 A1) oder bei Verlustkörnern (EP 2 742 791 A2) sowie eine optische Erfassung von geernteten Körnern und Auswertung zur Bestimmung der Tausendkornmasse unter Laboratoriumsbedingungen (DD 277 756 A1, DD 157 483 A1, DE 297 09 234 U1, DE 196 45 068 C1, CN 110132384 A , CN 112 304 947 A und W. Wu et al., GainTKW: A Measurement System of Thousand Kernel Weight Based on the Android Platform, Agronomy 2018, 8, 178) beschrieben.

Bisherige Messungen von Stärke- und Proteingehalten und Tausendkorngewichten von bestimmten Weizenzüchtungen haben keine nennenswerte Korrelation zwischen diesen Größen ergeben (Dobre, P.S. et al, Protein content, thousand kernel weight (tkw) and volumetric mass (vm) variability in a set of wheat mutated and mutated/recombinant dh lines, AgroLife Scientific Journal, Volume 5, Nr. 1, 2016, Seiten 59-62), was analog auch für den Zusammenhang zwischen Protein- und Stärkegehalt einerseits und Dichte und Tausendkorngewicht andererseits bei Mais gilt (Hilliard, J. et al., Starch content, test weight, and other quality parameters of corn produced in different maturity areas of Ontario, Crop Science Vol. 14,4 (1974), Seiten 546 bis 548).

### Aufgabe

Alle diese Vorgehensweisen sind demnach nicht geeignet, um eine oder mehrere der besagten massenspezifischen Größen (Tausendkornmasse, Dichte, Abmessungen der Körner) oder eine andere Information, aus welcher eine oder mehrere der besagten Größen abgeleitet werden könnte, auf eine relativ einfache Weise zu ermitteln.

Es ist eine Aufgabe der vorliegenden Erfindung, ein gegenüber dem Stand der Technik verbessertes Verfahren und eine Anordnung zur Messung einer massen- oder größenspezifischen Größe der Körnerfrüchte bei der Körnerernte bereitzustellen, das bzw. die eine detailliertere Information über das Erntegut als bisher ermöglicht. Ebenso ließe sich damit eine Aussage über die passende Aussaatmenge oder richtige Düngermengen der Frühjahrsdüngung ermöglichen.

### Lösung

Diese Aufgabe wird durch die Lehren der Ansprüche 1, 9 und 10 gelöst, wobei die untergeordneten Ansprüche vorteilhafte Ausführungsformen beschreiben.

Ein Verfahren und eine Anordnung zur Messung des Tausendkorngewichts von Körnern von Körnerfrüchten umfassen folgende Schritte oder Mittel zur Durchführung:
Aufnehmen eines Spektrums von zu untersuchenden Körnern mittels eines im Nahinfrarotbereich arbeitenden Spektrometers,
Ermitteln der Gehalte der untersuchten Körner an Inhaltsstoffen anhand des Spektrums, wobei die Inhaltsstoffe Wasser, Protein und Stärke oder damit korreliert sind; und
Ableiten des Tausendkorngewichts anhand der ermittelten Gehalte der Körner an Inhaltsstoffen.

Mit anderen Worten wird das Tausendkorngewicht anhand von Spektren der Körnerfrüchte, die im Folgenden als Körner oder Korn bezeichnet werden, ermittelt. Weitere Einzelheiten dazu werden in der Figurenbeschreibung diskutiert.

Hierbei wird anhand des Spektrums der Gehalt der Körnerfrüchte an Inhaltsstoffen ermittelt und anhand des ermittelten Gehalts das Tausendkorngewicht bestimmt. Diese Inhaltsstoffe sind Wasser, Protein und Stärke oder damit korreliert, wie es beispielsweise bei dem mit Protein korrelierten Gluten der Fall ist.

Die massen- und/oder größenspezifische Größe kann während der Ernte der Körnerfrüchte mittels einer Erntemaschine fortlaufend an jeweils anderem Erntegut ermittelt und georeferenziert in einer Karte aufgezeichnet werden. Es ist aber auch eine mobile oder stationäre Anwendung des Verfahrens und der zugehörigen Vorrichtung an beliebiger Stelle möglich. Zusätzlich kann wenigstens ein Inhaltsstoff des Ernteguts gemessen und gemeinsam mit der massen- und/oder größenspezifischen Größe in der Karte georeferenziert abgespeichert werden. Zusätzlich kann der Ertrag teilflächenspezifisch gemessen und gemeinsam mit der massen- und/oder größenspezifischen Größe in der Karte georeferenziert abgespeichert werden. Die Karte kann zur Planung agronomischer Maßnahmen verwendet werden.

### Ausführungsbeispiel

Diese und andere Aufgaben, Merkmale und Vorteile der vorliegenden Erfindung werden dem Fachmann nach dem Lesen der folgenden detaillierten Beschreibung und angesichts der Zeichnungen offensichtlich. Es zeigt:
- Fig. 1: eine schematische seitliche Ansicht einer Erntemaschine mit einer Messanordnung zur spektroskopischen Untersuchung eines Erntegutstroms,
- Fig. 2: eine vergrößerte seitliche Ansicht des Auslassbereichs des Körnerelevators der Erntemaschine aus Figur 1,
- Fig. 3: ein Diagramm, in dem ein Zusammenhang zwischen Tausendkorngewicht (TKW) und Proteingehalt für eine Anzahl an Messwerten eingetragen ist,
- Fig. 4: ein Diagramm, in dem ein Zusammenhang zwischen Tausendkorngewicht (TKW) und Stärkegehalt für eine Anzahl an Messwerten eingetragen ist,
- Fig. 5: ein Diagramm, in dem ein Zusammenhang zwischen gemessenen und anhand von Spektren ermittelten Tausendkorngewichten (TKW) eingetragen ist,
- Fig. 6: ein Flussdiagramm hinsichtlich der Vorgehensweise beim Messen des Tausendkorngewichts, und
- Fig. 7: ein Flussdiagramm zur Auswertung des gemessenen Tausendkorngewichts.

Die vorliegende Erfindung bezieht sich auf die Ermittlung des Tausendkorngewichts (und optional der Massendichte, z.B. Hektolitergewicht, und/oder der Abmessung von Körnern) anhand einer spektroskopischen Messung. Im Ausführungsbeispiel erfolgt diese Messung auf einer Erntemaschine, was eine Kartierung der Messwerte erlaubt. Es wäre jedoch auch eine Messung mit einem mobilen oder stationären Spektrometer z.B. in einem Labor, in oder an einem Transportfahrzeug oder einer Lagerstätte für Getreide möglich.

### Erntemaschine

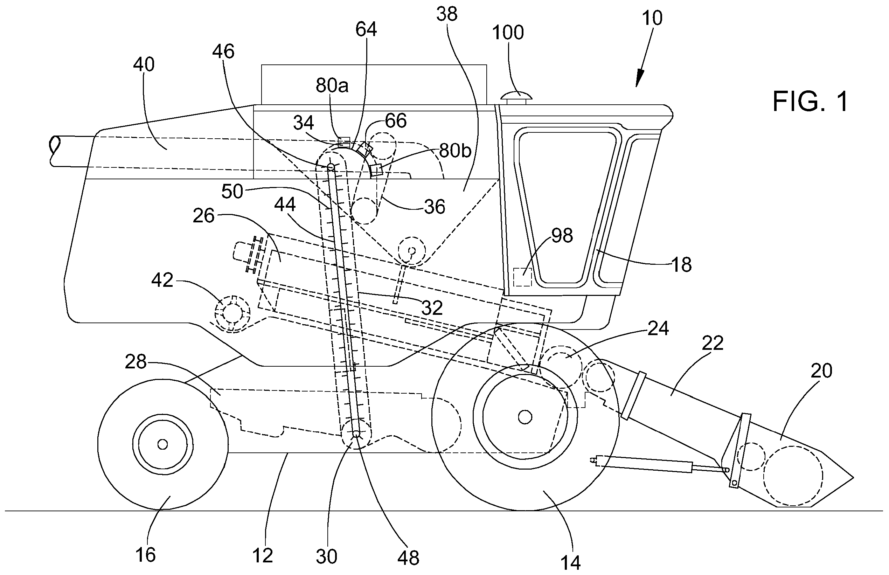

Die Figur 1 zeigt eine selbstfahrende landwirtschaftliche Erntemaschine 10 in Form eines Mähdreschers mit einem Rahmen 12, an dessen beiden Seiten im Eingriff mit dem Boden befindliche vordere Räder 14, die zum Vortrieb der Erntemaschine 10 in einer Vorwärtsrichtung dienen, die in der Figur 1 nach rechts verläuft, sowie rückwärtige, lenkbare Räder 16 angebracht sind. Der Betrieb der Erntemaschine 10 wird von der Bedienerkabine 18 aus kontrolliert. Im Erntebetrieb wird ein Schneidwerk 20 verwendet, um Korn enthaltendes Erntegut zu ernten und es einem Schrägförderer 22 zuzuführen. Das geerntete Gut wird durch den Schrägförderer 22 einer Leittrommel 24 zugeführt, welche das Erntegut einer axialen Erntegutbearbeitungseinrichtung 26 zuführt. Im Folgenden beziehen sich Richtungsangaben, wie vorn und hinten, auf die Vorwärtsrichtung der Erntemaschine 10.

Die Erntegutbearbeitungseinrichtung 26 umfasst ein Rotorgehäuse und einen darin angeordneten Rotor, an dem Gutbearbeitungselemente befestigt sind. Anstelle einer axialen Erntegutbearbeitungseinheit 26 kann auch eine tangentiale Dreschtrommel und eine ihr folgende axiale Trenneinrichtung oder Strohschüttler verwendet werden. Korn und Spreu, die durch einen Dreschkorb und ein Trennrost fallen, werden einem Reinigungssystem 28 mit einem Gebläse und in eine Schwingbewegung versetzbaren Lamellensieben zugeführt. Das Reinigungssystem 28 entfernt die Spreu und führt das saubere Korn über einen Schneckenförderer 30 einem Elevator 32 für sauberes Korn zu, welcher das saubere Korn in ein Übergangsgehäuse 34 fördert, aus dem es mittels eines weiteren Schneckenförderers 36 in einen Korntank 38 gefördert wird. Das saubere Korn im Korntank 38 kann durch einen Entladeschneckenförderer 40 auf einen Kornwagen, Anhänger oder Lastwagen entladen werden. Ausgedroschenes, die Erntegutbearbeitungseinrichtung 26 verlassendes Stroh wird durch einen Auslass aus der Erntegutbearbeitungseinrichtung 26 ausgestoßen und einer Auswurftrommel 42 zugeführt, die das Stroh nach hinten auswirft oder einem Strohhäcksler (nicht gezeigt) zuführt.

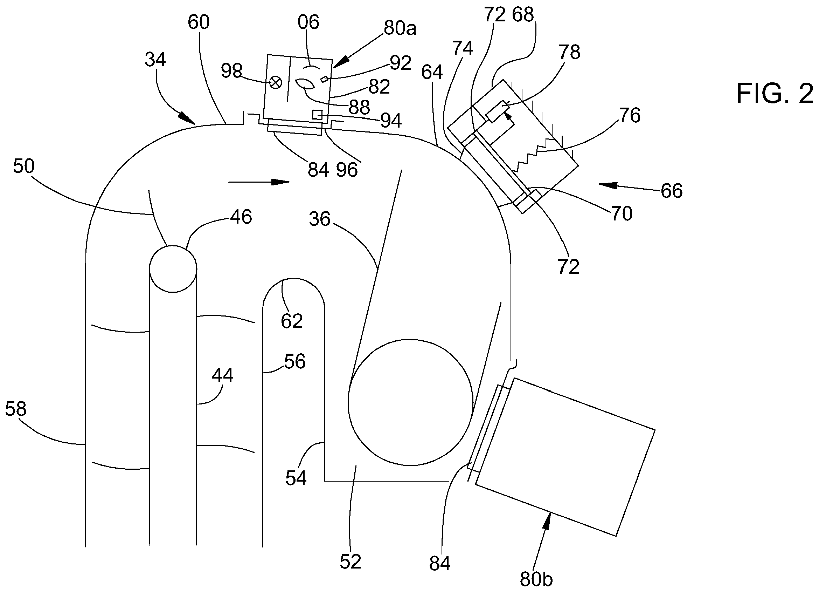

Die Figur 2 zeigt das Übergangsgehäuse 34 in einer vergrößerten Darstellung. Der Elevator 32 ist als Paddelförderer ausgebildet und umfasst eine oder mehrere Ketten 44, die um ein oberes Umlenkrad 46 und ein unteres Umlenkrad 48 umlaufen, von denen eines angetrieben ist. Die Kette 44 trägt mehrere schaufelförmige Paddel 50, die das nach oben heran geförderte Korn oberhalb des oberen Umlenkrads 46 näherungsweise horizontal abgeben. Das Übergangsgehäuse 34 umfasst eine Wanne 52, in der sich der Einlass des weiteren Schneckenförderers 36 befindet, und die durch eine Wand 54 nach unten und zum Elevator 32 hin begrenzt wird. Der seitliche Teil der Wand 54 ist durch einen dachförmigen Abschnitt 62 mit einer rückwärtigen Wand 56 eines Gehäuses des Elevators 32 verbunden, der nach vorn durch eine vordere Wand 58 eingeschlossen wird. Die vordere Wand 58 geht an ihrer Oberseite kurvenförmig in einen Deckel 60 des Übergangsgehäuses 34 über. An der dem Auslass des Elevators 32 gegenüber liegenden Seite wird das Übergangsgehäuse 34 durch eine konkav gekrümmte Prallplatte 64 einer Durchsatzermittlungseinrichtung 66 nach oben und vorn begrenzt, gegen die das vom Elevator 32 abgeworfene Korn prallt.

Die Durchsatzermittlungseinrichtung 66 umfasst weiterhin ein an der Außenseite der Prallplatte 64 und des Übergangsgehäuses 34 positioniertes Gehäuse 68, das starr mit dem Rahmen 12 verbunden ist. In dem Gehäuse 68 ist eine Platte 70 durch Führungen 72 in einer schräg nach oben und vorn verlaufenden Richtung verschiebbar gelagert. Die Platte 70 ist durch ein Rohr 74 starr mit der Prallplatte 64 verbunden und kann sich mit der Prallplatte 64 und dem Rohr 74 gegenüber dem Gehäuse 68 bewegen. Eine Feder 76 spannt die Platte 70 nach unten und hinten vor, so dass die Platte 70 durch auf die Prallplatte 64 auftreffendes Korn gegen die Kraft der Feder 76 nach oben und vorn bewegt wird. Ein Positionssensor 78 in Form eines Potentiometers erfasst die Position der Platte 70 und demnach der Prallplatte 64, so dass sein Ausgangssignal ein Maß für den Massendurchsatz des vom Elevator 32 abgegebenen Erntegutstroms ist.

### Spektrometer

Weiterhin sind in den Figuren 1 und 2 an zwei unterschiedlichen Positionen Spektrometer 80a, 80b eingezeichnet. In der Regel wird nur eines der Spektrometer 80a oder 80b eingebaut. Die beiden eingezeichneten Spektrometer 80a, 80b dienen zur Veranschaulichung verschiedener Positionierungsmöglichkeiten. Die Spektrometer 80a, 80b sind identischen Aufbaus, der im Folgenden anhand des Spektrometers 80a diskutiert wird. Das Spektrometer 80a umfasst ein Gehäuse 82 mit einer Öffnung, in der ein Fenster 84 angeordnet ist, dessen Scheibe vorzugsweise aus Saphirglas oder einem anderen, hinreichend verschleißresistenten und im Wellenlängenbereich des bei der Untersuchung verwendeten Lichts ausreichend transparentem Material besteht. Innerhalb des Gehäuses 82 befindet sich eine Lichtquelle 86, die den vom Elevator 32 abgegebenen Erntegutstrom durch das Fenster 84 mit breitbandigem, so genanntem weißem Licht bestrahlt, das in der Regel den Nahinfrarotbereich abdeckt. Vom Erntegutstrom reflektiertes Licht tritt durch das Fenster 84 wieder in das Gehäuse 82 ein, wird dort durch ein Linsensystem 88 auf ein dispersives Element 90 in Form eines konkaven Spiegels mit einer an seiner Unterseite angebrachten Gitterstruktur geleitet, die das Licht in von der Wellenlänge abhängige Richtungen ablenkt und erreicht schließlich einen Detektor 92 mit einer Reihe lichtempfindlicher Elemente, die von der Intensität des empfangenen Lichts abhängige Signale abgeben. Das hier beschriebene Verfahren ist nur eine mögliche Ausführungsform einer Spektrometeranordnung zur Messung von Inhaltstoffen in landwirtschaftlichen Gütern. Es ist auch möglich, dass die Lichtquelle gegenüber dem Fenster 84 angeordnet wird, so dass Licht, das das Erntegut durchleuchtet, in auf das Linsensystem trifft. Es ist auch möglich in einer Kugelanordnung zu messen (s. WO 2019/14997 A1). Das dispersive Element kann auch andersartig ausgeführt werden, z.B. als ein Transmissionsgitter oder es kann ein MEMS sein. Eine mit dem Detektor 92 verbundene Auswertungseinrichtung 94 wertet die Ausgangssignale des Detektors 92 aus und liefert Spektren und/oder daraus abgeleitete Informationen, z. B. Anteile an Inhaltsstoffen im Erntegutstrom. Geeignete Spektrometer werden in der DE 199 22 867 A1 und in der DE 10 2004 048 103 A1 beschrieben, deren Offenbarungen durch Verweis mit in die vorliegenden Unterlagen aufgenommen werden.

Das Spektrometer 80a ist oberhalb des Deckels 60 des Übergangsgehäuses 34 angeordnet, wobei sich das Fenster 84 innerhalb einer Öffnung in einer Rampe 96 befindet, die unmittelbar stromauf der Prallplatte 64 angeordnet ist. Die Rampe 96 dient dazu, den vom Elevator 32 abgegebenen Erntegutstrom auf die Prallplatte 64 zu lenken und insbesondere zu verhindern, dass Korn an der bezüglich des Erntegutstroms vorderen Kante der Prallplatte 64 in unerwünschter Weise an deren Außenseite gelangt. Dank einer Anstellung der Rampe 96 und des Fensters 84 um einen relativ kleinen Winkel, der beispielsweise zwischen 3 und 5° betragen kann, wird durch den Massenfluss des Ernteguts eine selbstreinigende Funktion der Verglasung bzw. Scheibe des Fensters 84 des Spektrometers 80a erzielt. Einer Verschmutzung bzw. Ansammlung von Ablagerungen von Erntegutresten oder anderen Schmutzpartikeln auf der Scheibe des Fensters 84 wird demnach entgegengewirkt.

Das Spektrometer 80b ist hingegen am stromab liegenden Ende der Prallplatte 64 angeordnet, wobei sich das Fenster 84 schräg nach unten und hinten zur Mitte der Wanne 52 erstreckt. Die Fenster 84 beider Spektrometer 80a, 80b werden vom Erntegutstrom umspült, so dass eventuelle Verunreinigungen mitgerissen werden und nicht an den Fenstern 84 anhaften.

Die Auswertungseinrichtung 94 des Spektrometers 80a oder 80b und der Positionssensor 78 der Durchsatzermittlungseinrichtung 66 sind über ein Bussystem oder ein zugeordnetes Kabel oder über Funk oder optisch mit einer Aufzeichnungseinrichtung 98 verbunden, die sich in der Bedienerkabine 18 befindet, vgl. Figur 1. Die Aufzeichnungseinrichtung 98 ist weiterhin mit einer Positionsbestimmungseinrichtung 100 in Form einer Antenne und Empfangseinrichtungen zum Empfang und zur Verarbeitung von Signalen eines satellitenbasierten Positionsbestimmungssystems, z. B. GPS und/oder Glonass und/oder Eureka, verbunden. Die Signale der Auswertungseinrichtung 94 des Spektrometers 80a oder 80b und des Positionssensors 78 der Durchsatzermittlungseinrichtung 66 werden durch die Aufzeichnungseinrichtung 98 demnach georeferenziert aufgezeichnet, um sie später zu Abrechnungszwecken oder für die Verwendung in der Präzisionslandwirtschaft nutzen zu können. Außerdem können diese Signale zur selbsttätigen Einstellung von Komponenten der Erntemaschine 10 verwendet werden, beispielsweise zur Einstellung der Gebläsedrehzahl und Sieböffnungsweite des Reinigungssystems 28 oder der Geschwindigkeit der axialen Erntegutbearbeitungseinrichtung 26. Die Anbringung des (oder der) Spektrometer(s) 80a und/oder 80b und der Durchsatzermittlungseinrichtung 66 in unmittelbarer Nachbarschaft hat den Vorteil, dass jeweils Messwerte desselben Ernteguts erfasst werden, d.h. dass die zeitliche Korrelation der Messwerte sehr gut ist. Es wäre jedoch auch möglich, das Spektrometer 80a oder 80b an beliebiger anderer Stelle der Erntemaschine 10 anzubringen, z.B. am Elevator 32 oder einer durch den Elevator 32 befüllbaren, separaten Messkammer (vgl. CA 2 182 989 C oder EP 1 305 994 A1) oder am Schneckenförderer 30.

### Auswertung der Spektren

Das Ziel der vorliegenden Erfindung liegt darin, eine massen- und/oder größenspezifische Größe des geernteten Korns zu ermitteln, zum Beispiel dessen Tausendkorngewicht, Massendichte und/oder Abmessungen. Die Größe (Abmessungen, d.h. Länge und/oder Durchmesser und/oder Volumen) der Körner und die in kg oder g gemessene Masse oder das in N (Newton) gemessene Gewicht der einzelnen Körner variiert nicht nur abhängig von der Art des Ernteguts, sondern auch abhängig vom jeweiligen Standort, denn je nach Zufuhr von Wasser, Licht, Bodeneigenschaften, Bestandsdichte, Düngung etc. werden die Körner ortsabhängig größer oder kleiner. Zudem wachsen, abhängig von lokalen Bedingungen, mehr oder weniger Pflanzen mit mehr oder weniger Körnern daran. Dadurch variieren auch der Ertrag, der sich aus Produkt der Anzahl der Körner je Flächeneinheit mit deren jeweiliger Masse berechnen lässt. Für agronomische Zwecke ist es daher interessant, eine Karte hinsichtlich einer von der Masse und/oder Größe der Körner abhängigen Größe zu erhalten, basierend auf welcher die zeitlich nachfolgenden Maßnahmen ortsspezifisch geplant werden können. Die besagte Größe ist insbesondere die Tausendkornmasse, die eine agronomisch gebräuchliche Größe darstellt.

Im Stand der Technik ist eine Messung von Inhaltsstoffen (Protein- und Stärkegehalt) in Korn bereits bekannt und etabliert. Hierzu wird üblicherweise die Nahinfrarotspektroskopie verwendet. Dabei wird der relative Feuchte-, Stärke- oder Proteinanteil (prozentual) in der Trocken- oder Gesamtmasse einer Probe gemessen. In der Auswertungseinrichtung 94 des Spektrometers 80a und/oder 80b sind dazu Kalibrierdaten hinterlegt, anhand welcher die gemessenen Spektren in die besagten Inhaltsstoffanteile umgerechnet werden. Diese Auswertung (auch hinsichtlich der anderen agronomischen Daten, die im Folgenden diskutiert werden) kann auch in der Aufzeichnungseinrichtung 98 oder einem beliebigen anderen Rechner auf der Erntemaschine 10 oder einem davon beabstandeten Rechner erfolgen, an den die Spektren drahtlos übertragen werden, oder die Spektren werden zunächst (insbesondere georeferenziert) aufgezeichnet und später auf einem beliebigen Rechner ausgewertet.

Da das Körnergewicht ein struktureller Parameter und keine chemische Größe ist, kann man prima facie nicht davon ausgehen, dass das Tausendkorngewicht oder eine verwandte agronomische Größe (insbesondere die Massendichte bzw. das Hektolitergewicht des Korns oder dessen Abmessungen) mittels eines Nahinfrarotspektrometers gemessen werden könnte (vgl. die Messungen von Dobre et al. und Hilliard et al., a.a.O).

Nähere Betrachtungen zeigen hingegen in überraschender Weise, dass aufgrund der Physiologie des Pflanzenwachstums dennoch eine Abschätzung des Korngewichts anhand von Inhaltsstoffwerten möglich ist und somit das Korngewicht zumindest näherungsweise basierend auf Inhaltsstoffsensoren erfasst werden kann. Der Grund dafür liegt darin, dass beispielsweise Weizenkörner innere Proteinkerne ähnlicher Größe bilden. Der Stärkekörper wächst um die Proteinkerne herum und seine endgültige Größe hängt vom Ernährungszustand der individuellen Pflanze ab. Aus diesem Grunde hat ein schweres und großes Korn typischerweise ein hohes Verhältnis aus Stärke zu Protein und ein leichtes und kleines Korn ein niedriges Verhältnis von Stärke zu Protein.

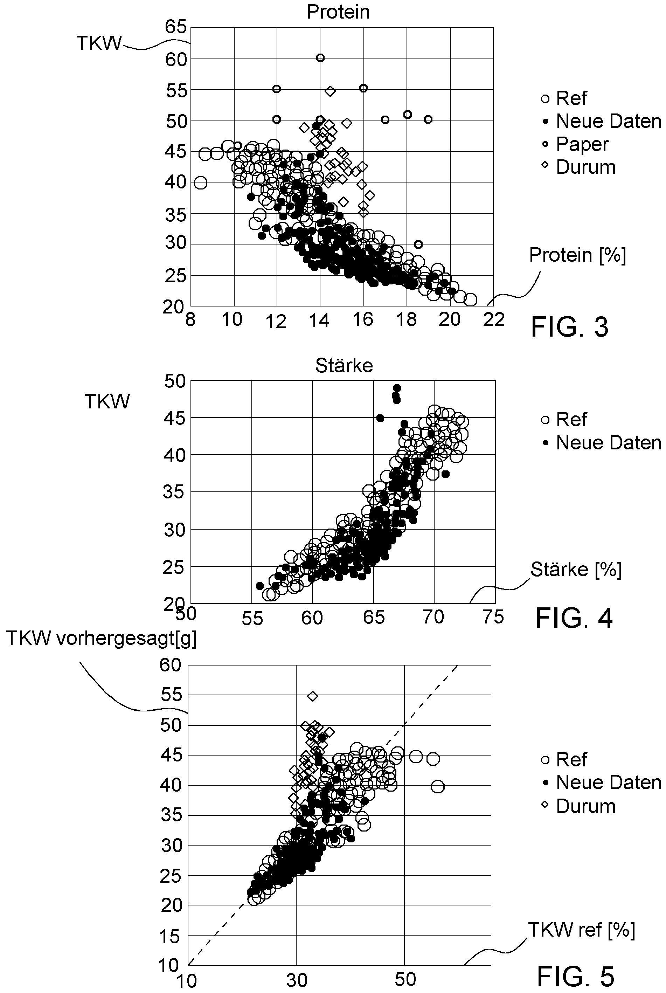

Hierzu sei auf die Figuren 3 und 4 verwiesen, in denen für verschiedene Proben Messwerte für Proteingehalt bzw. Stärkegehalt und Tausendkorngewicht (TKW) eingetragen sind. Die unterschiedlichen Punkte repräsentieren eine Referenzmessung (d.h. eine Anzahl an Messwerten einer bestimmten Weizenzüchtung, die zur Erstellung von Kalibrierdaten genutzt werden), neue Daten für dieselbe Züchtung (später ermittelt), sowie Daten von Durum-Weizen und einige Messwerte aus Dobre et al., a.a.O. Es ist erkennbar, dass bei Messungen innerhalb einer Züchtung oder sogar innerhalb von Getreide ähnlicher Sorte gewisse Zusammenhänge erkennbar sind. So sinkt das Tausendkorngewicht mit steigendem Proteingehalt, während es mit steigendem Stärkegehalt ansteigt. Bei den Messungen für Durum und jenen von Dobre et al. wären andere Koeffizienten bei der Auswertung sinnvoll.

Durch mathematische Verfahren wurde anhand der Referenzmessung eine geeignete Gleichung erstellt, mit welcher das Tausendkorngewicht (TKW) modelliert werden kann: $TKW ∼ c 0 + c 1 * Stärke \left[%\right] / Protein \left[%\right] + c 2 * Feuchte \left[%\right] / \left(Protein\left[%\right]*\left(1-Feuchte\left[%\right]/100\right)\right.$

Eine entsprechende Kurve ist in der Figur 5 gezeigt, in der gemessene und anhand der Gleichung (1) gemessene Tausendkorngewichte verglichen werden. Die dazu verwendeten Koeffizienten c0, c1 und c2 wurden mit dem Referenzmaterial bestimmt und auf Proben gleicher Kornzüchtung (neue Daten) angewendet. Für die Referenzmessung und die neuen Daten ergibt sich eine brauchbare Korrelation. Es besteht demnach die Möglichkeit, anhand der mit dem Spektrometer 80a oder 80b gemessenen Spektren das Tausendkorngewicht zu bestimmen. Durum ist eine andere Züchtung, wie auch das in dem Paper von Dobre et al. untersuchte Getreide. Der Plot zeigt, dass die Koeffizienten c0, c1 und c2 für Durum ungeeignet sind und entsprechende, angepasste Koeffizienten noch bestimmt werden müssen.

Der Protein- und Stärkegehalt werden demnach durch den Inhaltsstoffsensor (Spektrometer 80a, 80b) bezüglich der Trockenmasse ermittelt und der Feuchtegehalt bezüglich der Gesamtmasse bestimmt. Die Konstanten c0, c1 und c2 können für eine jeweils zu untersuchende Züchtung von Korn durch Kalibriermessungen bestimmt werden. Sie sind für unterschiedliche Züchtungen unterschiedlich und es kann sich bei anderen Züchtungen bzw. Arten von Korn auch ergeben, dass andere Gleichungen zu verwenden sind.

Die Relation zwischen dem Tausendkorngewicht und Inhaltsstoffen kann, anstelle die Gleichung (1) zu verwenden, auch durch generellere Funktionen modelliert oder erlernt werden, wie neuronale Netzwerke, oder das Tausendkorngewicht könnte direkt aus den NIR-Spektren ermittelt werden (analog zur bisherigen Vorgehensweise bei der Bestimmung der Inhaltsstoffe anhand der Spektren durch Kalibrierdatenerstellung).

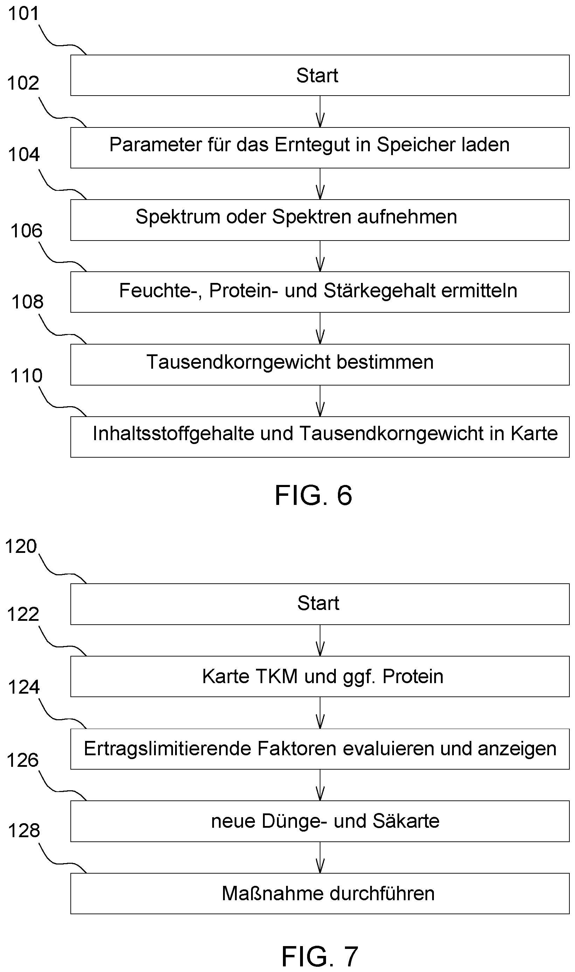

Eine mögliche Vorgehensweise zur Messung und Kartierung des Tausendkorngewichts ist in der Figur 6 gezeigt. Nach dem Start im Schritt 101 werden im Schritt 102 die Parameter für das jeweils geerntete Erntegut (Kornzüchtung) in einen Speicher der Auswertungseinrichtung 94 geladen. Die Parameter umfassen Kalibrierdaten zur Umrechnung der Spektren in Feuchte-, Protein- und Stärkegehalte und die Konstanten c0, c1 und c2 von Gleichung (1). Im Schritt 104 wird ein Spektrum oder mehrere Spektren aufgenommen, im Schritt 106 wird der Feuchte-, Protein- und Stärkegehalt anhand des Spektrums oder der Spektren und der Kalibrierdaten ermittelt, und im Schritt 108 das Tausendkorngewicht (TKW) anhand der Gleichung (1) bestimmt. Im Schritt 110 werden die Inhaltsstoffgehalte und das Tausendkorngewicht ortsspezifisch in eine Karte eingetragen, unter Verwendung von Positionssignalen der Positionsbestimmungseinrichtung 100. Auf den Schritt 110 folgt wieder der Schritt 104, es sei denn, der Vorgang ist beendet, wenn das Feld abgeerntet ist. Die Ermittlung und Kartierung der Tausendkornmasse können auch im Nachgang, z.B. in einem Farm-Management-Informations-System, erfolgen. Dazu können Protein, Stärke und Feuchte (oder die Spektren) kartiert und später ausgewertet werden.

Im Schritt 108 kann in an sich bekannter Weise eine Referenzierung des ermittelten Tausendkorngewichts auf den Ort erfolgen, an dem die Körner gewachsen sind, vgl. EP 3 008 990 A2, d.h. die Laufzeit des Ernteguts zwischen Ernte und Sensierung und der inzwischen zurückgelegte Weg der Erntemaschine 10 bzw. Positionsbestimmungseinrichtung 100 kompensiert werden.

Zudem kann der Ertrag (in Volumen oder Masse je Flächeneinheit) mittels der Durchsatzermittlungseinrichtung 66 (s. hierzu auch EP 3 901 588 A1 und den dort zitierten Stand der Technik) gemessen und kartiert werden und anhand des Ertrags und des Tausendkorngewichts die Massendichte (Hektolitergewicht o.ä.) des Korns errechnet werden. Alternativ oder zusätzlich kann anhand des Ertrags und des Tausendkorngewichts die Anzahl der Körner je Flächeneinheit des Felds bestimmt werden. Hierzu sei auf die ältere Patentanmeldung DE 10 2022 110 185.1 verwiesen, deren Offenbarung durch Verweis mit in die vorliegenden Unterlagen aufgenommen wird.

Das Tausendkorngewicht ist auch mit der Dichte (Hektolitergewicht) korreliert, sodass auch letztere durch ein angepasstes Modell anhand der Spektren oder Inhaltsstoffe ermittelt werden könnte. Analog enthalten die Spektren aufgrund der oben diskutierten Zusammenhänge zwischen Inhaltsstoffen und Abmessungen der Körner auch eine Information über die Abmessungen. Diese können demnach, analog zur Vorgehensweise bei dem Tausendkorngewicht, anhand der Spektren ermittelt werden, entweder über den Umweg mit den Inhaltsstoffen oder direkt anhand der Spektren durch entsprechende Kalibrierdaten.

### Weitere Verwendung des Tausendkorngewichts

Die gemäß der Vorgehensweise der Figur 6 ermittelte Karte kann per Datenfernübertragung oder über ein tragbares Speichermittel an einen stationären oder mobilen Rechner eines Landwirts oder Agronomen übersandt und zur Planung agronomischer Maßnahmen verwendet werden. Hierbei kann die Maßnahme basierend auf der Masse der Körner und/oder der Anzahl an Körnern je Flächeneinheit und/oder deren Abmessungen (die jeweils anhand der anderen Größen und des Ertrags berechnet oder im Fall der Abmessungen, wie im vorhergehenden Absatz beschrieben, direkt anhand der Spektren ermittelt werden können) geplant werden. Insbesondere kann die Särate basierend auf der Anzahl an Körnern je Flächeneinheit und/oder deren Abmessungen und/oder deren Masse geplant werden.

Die Figur 7 zeigt eine mögliche Vorgehensweise zur Nutzung der mit den Verfahren nach Figur 6 erzeugten Karte. Nach dem Start im Schritt 120 wird im Schritt 122 die Karte auf einem beliebigen Rechner aufgerufen, d.h. in dessen Speicher geladen. Der Rechner kann ein stationärer Rechner sein, der sich im Büro des Landwirts oder eines beauftragten Agronomen befindet, dessen Feld mit der Erntemaschine 10 nach der Vorgehensweise der Figur 6 abgeerntet wurde, oder der Landwirt oder beauftragte Agronom verwendet einen mobilen Rechner (Laptop, Tablet, Smartphone etc.). Die Karte kann im Schritt 122 auch auf einer Anzeigeeinrichtung zweidimensional angezeigt werden, wobei unterschiedliche Tausendkornmassen (oder eine beliebige andere, für die Abmessungen oder Masse oder das Gewicht der Körner oder deren Anzahl je Fläche repräsentative Größe) durch Farben oder anderweitig (Graustufen, Schraffierungen etc.) angezeigt werden. Auch können in der Karte ein oder mehrere Inhaltsstoffgehalte des Ernteguts eingetragen sein, die mit dem Nahinfrarotsensor 80a, 80b gemessen und ortsspezifisch kartiert wurden. Dieser eine oder mehrere Inhaltsstoffgehalte, z.B. der Proteingehalt des Ernteguts, können ebenfalls angezeigt werden. Analog kann mit einer oder mehreren anderen Eigenschaften des Felds, z.B. der Bodenart oder ortsspezifisch variierenden oder über das Feld gleichbleibenden Düngemittelgaben oder gemessenen Nährstoffen im Boden oder dem Ertrag (an Körnern oder Masse je Fläche) oder der Anzahl der jeweils ausgesäten Pflanzen je Flächeneinheit verfahren werden.

Im Schritt 124 können ertragslimitierende Faktoren ermittelt und angezeigt werden, unter Verwendung der besagten Karte und insbesondere der ortsspezifisch aufgezeichneten Tausendkornmassen oder einer oder mehreren beliebigen anderen, für die Abmessungen oder Masse oder das Gewicht der Körner oder deren Anzahl je Flächeneinheit repräsentativen Größe(n). Zudem kann der (bei der Vorgehensweise nach Figur 6 mit dem Durchsatzermittlungseinrichtung 66 ebenfalls gemessene und ortsspezifisch erfasste) Ertrag je Flächeneinheit angezeigt und/oder anhand der Tausendkornmassen zur Berechnung der Körneranzahl je Flächeneinheit verwendet werden.

Durch die Dokumentation der (Tausend-) Kornmasse und/oder des Kornvolumens ist man in der Lage zu definieren, an welcher Stelle des Felds sich der Ertrag in welcher Weise zusammengesetzt hat. So kann ein gegebener Ertrag auf einer größeren Anzahl kleinerer Körner je Fläche oder einer kleineren Anzahl größerer Körner je Fläche beruhen. Um diesen Sachverhalt näher auszuleuchten, kann beispielsweise eine Berechnung der Anzahl der Körner pro Fläche erfolgen, indem man den ebenfalls ortsspezifisch kartierten, in Einheiten von Masse je Fläche gemessenen Ertrag, der z.B. durch die Durchsatzermittlungseinrichtung 66 im Körnerelevator 32 gemessen werden kann, durch die kartierte (Tausend-) Kornmasse dividiert.

Durch die berechnete Anzahl der Körner je Fläche und/oder deren Abmessungen bzw. Masse können Rückschlüsse gezogen werden, ob beim Pflanzenanbau mögliches Potenzial verschenkt wurde. So deuten wenige, große Körner darauf hin, dass noch Potential für einen höheren Ertrag vorhanden ist. In diesem Fall könnte die Särate erhöht werden oder die Bestockung der Pflanze über ausreichende Frühjahrsdüngung beeinflusst werden, d.h. in Zukunft an der betreffenden Stelle mehr Pflanzen je Flächeneinheit angebaut werden. Andererseits deuten viele, kleine Körner (Schmachtkorn) darauf hin, dass zu wenig Wasser/Nährstoffe vorhanden sind, um die angebaute Anzahl der Pflanzen je Fläche zu ernähren. In diesem Fall müsste die Särate vermindert und/oder die Düngung und ggf. Wasserversorgung angepasst werden. Um derartige Sachverhalte einfach erkennen zu können, kann eine Karte angezeigt werden, in welcher die Tausendkornmasse geteilt durch die Anzahl der Körner je Flächeneinheit (oder der Kehrwert davon) eingetragen wird. Hohe Werte für Tausendkornmasse geteilt durch die Anzahl der Körner je Flächeneinheit würden zur Vergrößerung der Särate/Stärkung der Bestockung veranlassen und kleine Werte zur Verminderung.

Die beschriebene Erfassung und Kartierung der Tausendkornmasse und/oder Abmessungen der Körner und/oder Anzahlen der Körner je Flächeneinheit hilft somit dem Landwirt oder Agronomen bei der Anpassung der pflanzenbaulichen Maßnahmen an wechselnde Böden und Gegebenheiten.

Im Schritt 124 kann eine Karte mit den limitierenden Faktoren erstellt werden. In dieser Karte kann ortsspezifisch angezeigt werden, an welchen Stellen welche agronomische Maßnahme gefehlt hat oder verbessert werden kann. Insbesondere kann fehlender oder überschüssiger Dünger anhand des Proteingehalts ermittelt werden, fehlendes oder überschüssiges Wasser anhand der Korngröße und eine zu hohe oder geringe Särate/zu starke Triebbildung (Bestockung) anhand der Tausendkornmasse und der Anzahl der Körner je Fläche.

Es wird demnach im Schritt 126 eine neue Dünge- und Säkarte erstellt, in welche die Rückschlüsse aus dem Schritt 124 einfließen. Hierbei kann der Landwirt oder beauftragte Agronom anhand der angezeigten Daten festlegen, an welchen Stellen welche Maßnahmen sinnvoll sind, sei es qualitativ oder quantitativ. Hierbei kann jedoch auch ein rechnergestützter Algorithmus Verwendung finden, der insbesondere selbstlernend arbeitet, d.h. anhand vorangehender Maßnahmen und deren gemessener Auswirkung nach und nach lernt, welche Maßnahmen auf dem betreffenden Feld sinnvoll sind.

Die auf die beschriebene Weise erstellte Karte mit den ortsspezifisch durchzuführenden Maßnahmen wird im Schritt 128 bei einer nachfolgenden Maßnahme, insbesondere im nachfolgenden Anbauzyklus, berücksichtigt.

Nach alledem ist erkennbar, dass die ortsspezifisch erfassten und kartierten Gewichte der Körner anhand der Signale des Spektrometers 80a, 80b ermittelt werden können, und unter Hinzuziehung der Ertragswerte weitere agronomische Größen bestimmbar sind (Anzahl der Körner je Flächeneinheit und Massendichte, die aber auch anhand der Spektren gemessen werden kann). Diese agronomischen Größen bilden eine sinnvolle Grundlage für die Planung der nachfolgenden Maßnahmen, insbesondere die ortsspezifisch anzusteuernde Särate und eine angepasste Frühjahrsdüngung, um Kornzahl pro Fläche und Tausendkornmasse hinreichend zu beeinflussen und zum Ertragsziel zu bringen. Wie oben erläutert, können diese Größen aus einer beliebigen Auswahl aus folgenden gemessenen und kartierten Werten ermittelt werden: Ertrag, Abmessungen (oder Masse) der Körner und Anzahl der Körner je Flächeneinheit.

## Patentansprüche

1. Verfahren zur Messung des Tausendkorngewichts von Körnern von Körnerfrüchten, mit folgenden Schritten:
Aufnehmen eines Spektrums von zu untersuchenden Körnern mittels eines im Nahinfrarotbereich arbeitenden Spektrometers (80a, 80b),
Ermitteln der Gehalte der untersuchten Körner an Inhaltsstoffen anhand des Spektrums, wobei die Inhaltsstoffe Wasser, Protein und Stärke oder damit korreliert sind; und
Ableiten des Tausendkorngewichts der untersuchten Körner anhand der ermittelten Gehalte der untersuchten Körner an Inhaltsstoffen.

2. Verfahren nach Anspruch 1, wobei das Tausendkorngewicht während der Ernte der Körner mittels einer Erntemaschine (10) fortlaufend an jeweils anderem Erntegut ermittelt und georeferenziert in einer Karte aufgezeichnet wird.

3. Verfahren nach Anspruch 2, wobei die ermittelten Gehalte an Inhaltsstoffen gemeinsam mit dem Tausendkorngewicht in der Karte georeferenziert abgespeichert werden.

4. Verfahren nach Anspruch 2 oder 3, wobei zusätzlich der Ertrag teilflächenspezifisch gemessen und gemeinsam mit dem Tausendkorngewicht in der Karte georeferenziert abgespeichert wird, welche zur Planung agronomischer Maßnahmen verwendbar ist.

5. Anordnung zur Messung des Tausendkorngewichts von Körnern von Körnerfrüchten, umfassend:
ein im Nahinfrarotbereich arbeitendes Spektrometer (80a, 80b), das zum Aufnehmen eines Spektrums von zu untersuchenden Körnern der Körnerfrüchte konfiguriert ist, und
eine Auswertungseinrichtung (94), die konfiguriert ist:
die Gehalte der untersuchten Körner an Inhaltsstoffen anhand des Spektrums zu ermitteln, wobei die Inhaltsstoffe Wasser, Protein und Stärke oder damit korreliert sind; und
das Tausendkorngewicht anhand der ermittelten Gehalte der Körner an den Inhaltsstoffen abzuleiten.

6. Erntemaschine (10) mit einer Anordnung nach Anspruch 5.

## Claims

1. Method for measuring the thousand kernel weight of grains of grain crops, comprising the following steps:
recording a spectrum of the grains to be studied by means of a spectrometer (80a, 80b) operating in the near-infrared range,
determining the contents of ingredients of the studied grains on the basis of the spectrum, wherein the ingredients are water, protein and starch or are correlated therewith; and
deriving the thousand kernel weight of the studied grains on the basis of the determined contents of ingredients of the studied grains.

2. Method according to Claim 1, wherein the thousand kernel weight is determined continuously during harvesting of the grains by means of a harvester (10) on a different crop in each case and is recorded georeferenced in a map.

3. Method according to Claim 2, wherein the determined contents of ingredients are stored georeferenced in the map together with the thousand kernel weight.

4. Method according to Claim 2 or 3, wherein the yield is additionally measured subarea-specifically and is stored georeferenced in the map together with the thousand kernel weight, the map being usable for planning agronomic measures.

5. Arrangement for measuring the thousand kernel weight of grains of grain crops, comprising:
a spectrometer (80a, 80b) operating in the near-infrared range, which is configured to record a spectrum of grains of the grain crops to be studied, and
an evaluation device (94), which is configured:
to determine the contents of ingredients of the studied grains on the basis of the spectrum, wherein the ingredients are water, protein and starch or are correlated therewith; and
to derive the thousand kernel weight on the basis of the determined contents of the ingredients of the grains.

6. Harvester (10) comprising an arrangement according to Claim 5.

## Revendications

1. Procédé de mesure du poids de mille grains de grains de fruits à grains, avec des étapes suivantes :
enregistrement d'un spectre de grains à examiner au moyen d'un spectromètre proche infrarouge (80a, 80b),
détermination des teneurs des grains examinés en ingrédients à l'aide du spectre, les ingrédients étant de l'eau, des protéines et de l'amidon ou étant corrélés avec ceux-ci ; et
calcul du poids de mille grains des grains examinés à l'aide des teneurs déterminées des grains examinés en ingrédients.

2. Procédé selon la revendication 1, le poids de mille grains étant déterminé pendant la récolte des grains au moyen d'une moissonneuse (10) en continu sur un autre produit récolté respectivement et étant géoréférencé dans une carte.

3. Procédé selon la revendication 2, les teneurs déterminées en ingrédients étant sauvegardées conjointement avec le poids de mille grains de manière géoréférencée dans la carte.

4. Procédé selon la revendication 2 ou 3, le rendement étant mesuré en supplément de manière spécifique à la surface et étant sauvegardé conjointement avec le poids de mille grains de manière géoréférencée dans la carte, qui peut être utilisée pour la planification de mesures agronomiques.

5. Ensemble de mesure du poids de mille grains de grains de fruits à grains, comprenant :
un spectromètre proche infrarouge (80a, 80b), qui est configuré pour enregistrer un spectre de grains à examiner de fruits à grains, et
un dispositif d'évaluation (94), qui est configuré pour :
déterminer les teneurs des grains examinés en ingrédients à l'aide du spectre, les ingrédients étant de l'eau, des protéines et de l'amidon ou étant corrélés avec ceux-ci ; et
déduire le poids de mille grains à l'aide des teneurs déterminées des grains en ingrédients.

6. Moissonneuse (10) avec un ensemble selon la revendication 5.
